# EUROPEAN PATENT SPECIFICATION

(11) **EP 3 528 687 B1**
(45) Date of publication and mention of the grant of the patent: **29.01.2025**
(21) Application number: 17784311.7
(22) Date of filing: 19.10.2017
(51) Int. Cl.: A61B 1/00, A61B 1/12

(54) **METHOD AND SYSTEM FOR RECONDITIONING A MEDICAL INSTRUMENT, IN PARTICULAR AN ENDOSCOPE**
VERFAHREN UND SYSTEM ZUR WIEDERINSTANDSETZUNG EINES MEDIZINISCHEN INSTRUMENTS, INSBESONDERE EINES ENDOSKOPS
PROCÉDÉ ET SYSTÈME DE RECONDITIONNEMENT D'UN INSTRUMENT MÉDICAL, EN PARTICULIER D'UN ENDOSCOPE

(30) Priority: 19.10.2016 IT 201600105276
(43) Date of publication of application: 28.08.2019
(73) Proprietor: Steelco S.p.A., 31039 Riese Pio X (IT)
(72) Inventor: ZARDINI, Fabio, 31033 Castelfranco Veneto (IT)
(74) Representative: Petraz, Gilberto Luigi
(86) International application number: PCT/EP2017/076704
(87) International publication number: WO 2018/073339

(56) References cited:
- WO-A1-2009/143201
- WO-A1-2015/068131
- US-A1- 2012 031 506

## Description

### FIELD OF THE INVENTION

Embodiments described here concern a method and a system for reconditioning a medical instrument, in particular an endoscope.

The term "reconditioning", here and in the present description, generally refers to a complete cycle of treatment of a medical instrument, for example prewashing, washing, disinfecting, drying and storing the medical instrument.

### BACKGROUND OF THE INVENTION

As is known, tubular flexible medical instruments exist, made of various materials, in particular metal, plastic and/or rubber used in the medical field to explore, to examine using optical apparatuses or to empty the body's natural cavities, introduced through natural or artificial paths. Such endoscopic instruments may be, for example, colonoscopes, bronchoscopes, gastroscopes or other.

These flexible endoscopes allow to carry out check-ups and/or interventions on the patient in a non-invasive manner, acting from the inside of the body, thus avoiding having to intervene surgically from the outside.

Generally, an endoscope is formed by a control and service unit and an operating unit, connected by a flexible tube in which all the tubular elements or connecting channels between the two operating units are housed. In the most complex endoscopic instruments, there may be as many as eight connection channels, with functions for example for the conduction and suction of sterile or contaminated substances, housing of optical and surgical devices or other.

As can easily be imagined, normal endoscopic instruments are frequently used throughout the day inside structures such as hospitals, clinics, or suchlike. Therefore, before any new use, these instruments require a reconditioning process, in order to guarantee they are aseptic at the time of reuse.

Three main steps of reconditioning of an endoscopic instrument can be distinguished, which come between the last use and subsequent use: a first prewash step, a second washing and disinfection step and a third drying and storage step. Usually these three main steps are carried out in as many dedicated machines, so that for each step it is necessary to position the endoscopic instrument correctly in each specific machine.

For the instrument to be made suitably aseptic, each of the steps cited above must cover both the outside part and also the internal connection channels. To do this, in each of the three steps, the channels are connected, by means of corresponding connectors, to a corresponding processing machine, then to a machine that does the prewash, then to a machine that does the washing and disinfection, and finally to a machine which does the drying and storage. Normally, therefore, passing from one step to another and from one machine to another, the instrument must be handled so that the individual connectors of the individual connection channels are first connected and then disconnected.

As can be understood, the need for a prolonged and repetitive handling of the endoscopic instrument, essential to the reconditioning steps performed as described above, forces frequent and prolonged interventions by an operator. Because of these interventions, the medical instrument is also at high risk of re-contamination. The risk of re-contamination is difficult to overcome and reduce with known reconditioning machines and methods, as these are obligated process passages or steps.

For example, in document WO-A-2009/143201, an endoscope is described which is provided with a device for cleaning the site where the endoscopy is performed.

This document is therefore mainly intended to solve the problem of cleaning the site, or "lumen", where the endoscopy is performed, while the problem of contamination of the instrument is not posed, especially in steps of non-use, for example during the reconditioning steps of the endoscope.

Document US-A-2012/031506 concerns an apparatus for cleaning and disinfecting an endoscope.

This document takes into consideration a situation in which the endoscope is connected to a single apparatus to be cleaned and disinfected, but the problem of subjecting the endoscope to a reconditioning cycle comprising several reconditioning steps, typically three steps as described above, is not taken into consideration.

None of the above documents, WO-A-2009/143201 or US-A-2012/0031506, therefore poses the problem of contamination of a medical instrument, for example an endoscope, which is subjected to a plurality of reconditioning steps in which it may be subjected to contamination, in particular following its repeated handling, for example to transfer the medical instrument from one step to another.

US-A-2012/031506 discloses a system for reconditioning a medical instrument which comprises a device having a single connector configured to be removably connected to the medical instrument and suitable for being connected to counter-connectors of distinct dedicated reconditioning machines, said connector and said counter-connector being mating and a method to recondition a medical instrument which comprises a first step of univocally connecting a medical instrument to a connector, an intermediate step of reconditioning said medical instrument, in which said connector, with said medical instrument connected, is associated with a corresponding reconditioning counter-connector, said intermediate reconditioning steps of said medical instrument are carried out in succession, said method providing to keep said connector always connected to said medical instrument during the course of said one intermediate reconditioning step and also during the passage from one intermediate step to another, and, on each occasion, in relation to the intermediate reconditioning steps that follow each other, to connect/ disconnect said single specific connector to/from a respective counter-connector of the specific reconditioning machine in which a respective specific intermediate reconditioning step is carried out and a final step of univocally disconnecting said connector from said medical instrument once the reconditioning cycle has been completed, before said medical instrument is again used.

Other limitations and disadvantages of conventional solutions and technologies will be clear to a person of skill after reading the remaining part of the present description with reference to the drawings and the description of the embodiments that follow, although it is clear that the description of the state of the art connected to the present description must not be considered an admission that what is described here is already known from the state of the prior art.

There is therefore a need to provide a method and system for reconditioning a medical instrument, in particular an endoscope, which can overcome at least one of the disadvantages of the state of the art.

One purpose of the present invention is therefore to perfect a method for reconditioning a medical instrument, in particular an endoscope, in which the risk of contamination or re-contamination during the various reconditioning steps is drastically reduced compared to known reconditioning methods.

Another purpose of the present invention is to perfect a method for reconditioning a medical instrument, in particular an endoscope, in which the operations to transport and temporarily retrieve the medical instrument by the operators, during its reconditioning, which operations are generally carried out manually, are drastically simplified and unified, ensuring fast connection between the instrument and individual machines for each step of the reconditioning cycle.

Another purpose of the present invention is to perfect a method for reconditioning a medical instrument, in particular an endoscope, which dramatically reduces the possibility of positioning and recognition errors in the connection between the instrument and the machine for each of the steps of the reconditioning cycle.

Another purpose is to obtain an effective and functional system to implement said method, which also has an advantageously ergonomic shape and which preferably allows a single identification step of the connection between the instrument and the machine for each of the steps of the reconditioning cycle.

The Applicant has devised, tested and embodied the present invention to overcome the shortcomings of the state of the art and to obtain these and other purposes and advantages.

### SUMMARY OF THE INVENTION

The present invention is set forth and characterized in the independent claims, while the dependent claims describe other characteristics of the invention or variants to the main inventive idea.

In accordance with the above purposes and according to a first aspect of the invention, a system of reconditioning a medical instrument is as defined in annexed claim 1.

A reconditioning method of a medical instrument is according to annexed claim 4.

The method and system according to the present description therefore allow to isolate the medical instrument from any possible risk of contamination due to the repeated connections and disconnections of the connectors; indeed the single connector connected on the instrument side collects the connections at the terminals of the instrument. The same single specialized connector is used in the various reconditioning steps, since the same connection is identical in the counter-connectors of the reconditioning machines. The present invention therefore allows to carry out a single connection on the instrument side before the beginning of the reconditioning cycle, that is, upstream of the first intermediate reconditioning step, and a single disconnection at the end of the cycle, where for example the cycle can be concluded in a drying and storage/preservation apparatus or cabinet, from which the instrument is removed for a new use.

These and other aspects, characteristics and advantages of the present disclosure will be better understood with reference to the following description, drawings and attached claims. The drawings, which are integrated and form part of the present description, show some forms of embodiment of the present invention, and together with the description, are intended to describe the principles of the disclosure.

The various aspects and characteristics described in the present description can be applied individually where possible. These individual aspects, for example aspects and characteristics described in the attached dependent claims, can be the object of divisional applications.

It is understood that any aspect or characteristic that is discovered, during the patenting process, to be already known, shall not be claimed and shall be the object of a disclaimer.

### BRIEF DESCRIPTION OF THE DRAWINGS

These and other characteristics of the present invention will become apparent from the following description of some embodiments, given as a non-restrictive example with reference to the attached drawings wherein:
- fig. 1 is a three-dimensional view of a device provided with a connector and counter-connector, to carry out a reconditioning cycle of a medical instrument, in particular an endoscope, in a method according to embodiments described here;
- figs. 2a, 2b and 2c are schematic views of the main actuation steps of the present method to carry out a reconditioning cycle of a medical instrument, in particular an endoscope.

To facilitate comprehension, the same reference numbers have been used, where possible, to identify identical common elements in the drawings. It is understood that elements and characteristics of one embodiment can conveniently be incorporated into other embodiments without further clarifications.

### DETAILED DESCRIPTION OF SOME EMBODIMENTS

We shall now refer in detail to the various embodiments of the present invention, of which one or more examples are shown in the attached drawings. Each example is supplied by way of illustration of the invention and shall not be understood as a limitation thereof. For example, the characteristics shown or described insomuch as they are part of one embodiment can be adopted on, or in association with, other embodiments to produce another embodiment. It is understood that the present invention shall include all such modifications and variants.

Before describing these embodiments, we must also clarify that the present description is not limited in its application to details of the construction and disposition of the components as described in the following description using the attached drawings. The present description can provide other embodiments and can be obtained or executed in various other ways. We must also clarify that the phraseology and terminology used here is for the purposes of description only, and cannot be considered as limitative.

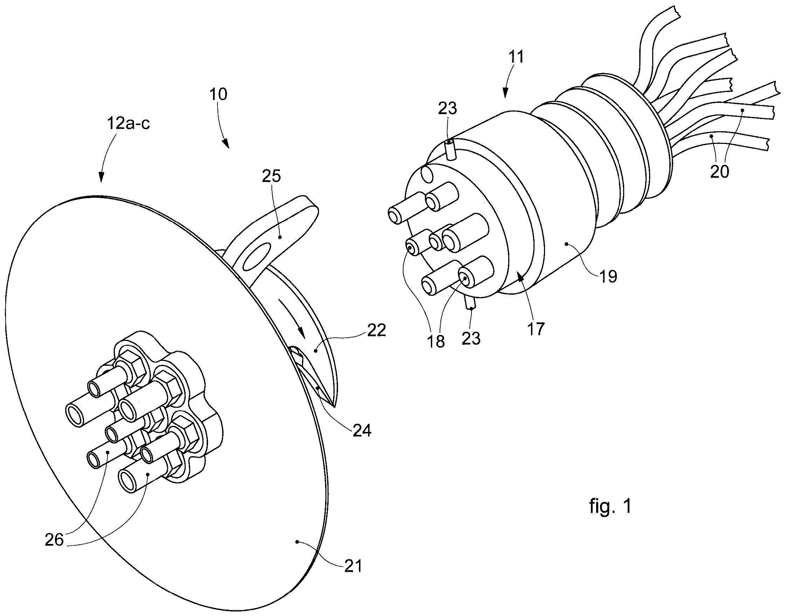

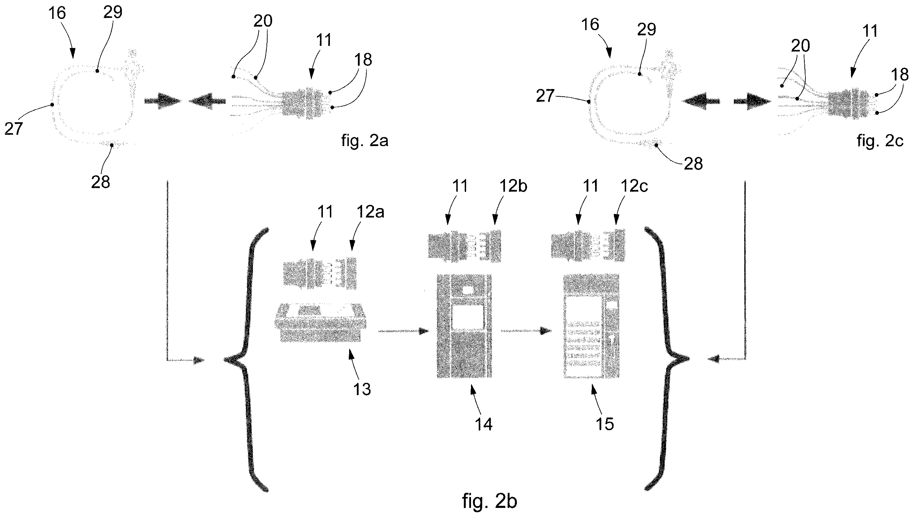

Fig. 1 is used to describe a device 10 for the treatment of a medical instrument 16, in particular, for example, but not limited to, a flexible endoscope, shown schematically in figs. 2a and 2c, usable in the embodiments described here in relation to a reconditioning cycle of the medical instrument 16.

Here and in the present description, a reconditioning cycle can be understood, for example, as a cycle that comprises, or consists of, a prewash step, a washing and disinfection step and a step of drying and storage/preservation/stocking of the medical instrument 16. Consequently, the beginning of the reconditioning cycle is represented by the beginning of the first prewash step, while the end of the reconditioning cycle may be understood, in the embodiments described here, to correspond to the moment when the medical instrument 16 is removed from its storage condition for a new use.

In some embodiments, the device 10 comprises a multiple connector 11, removable in itself and in relation to the medical instrument 16, and a multiple and fixed counter-connector 12a-c. The connector 11 and counter-connector 12a-c are coordinated so that they are correctly coupled to each other quickly and easily for an operator.

The connector 11 is a standardized multiple connector that has unified seatings to connect individual tubular elements, or flexible pipes 20, to coordinated apertures or terminals present in the medical instrument 16, for example an endoscope.

According to a variant, an intermediate connection element is provided, suitable to facilitate the correct connection of the connector 11 and counter-connector 12a-c.

The fixed counter-connector 12a-c can be installed on any reconditioning machine whatsoever that performs at least one of the steps of the treatment cycle of the medical instrument 16 discussed. With reference to figs. 1 and 2b, a counter-connector 12a of a first prewash machine 13, a counter-connector 12b of a second washing and disinfection machine 14 and a counter-connector 12c of a third drying and optionally storage machine 15 are shown schematically by way of example.

The counter-connectors 12a, 12b and 12c are unified and all coherent and mating with the connector 11 for a quick, reliable and secure connection.

The connector 11 comprises a connection head 17 provided with a series of connection elements 18.

A specific tubular treatment element 20 is connected to each of the connection elements 18, for example inside a casing 19 of the connector 11, in which tubular treatment element 20 a particular fluid for washing, disinfection or otherwise can flow.

Each of the tubular elements 20 is flexible and can be connected by means of suitable connection means to apertures or inlets of the medical instrument 16, so as to reach the channels and the internal zones.

As we said, the counter-connector 12a-c is installed on board each of the machines 13, 14 and 15, and in this regard comprises a support plate 21 which can be connected, for example, to one of the walls of the machine 13, 14, 15, in which the functions operable in the device 10 converge.

The connection head 17 of the connector 11 is inserted into a seating 22 of the counter-connector 12a-c, so as to obtain a stable and sealed connection.

The removable and sealed connection between the connection head 17 and the seating 22 can be carried out in any way whatsoever, for example by means of a rotating, bayonet or similar attachment system.

In this regard, in the connection head 17 of the connector 11, pins 23 can be provided by way of example, able to be inserted into corresponding slits 24, made in the seating 22 of the counter-connector 12a-c.

One of the slits 24 is visible in fig. 1. The other slit will be made on the seating 22 in a position diametrically opposite to the slit 24 visible in the drawing.

The seating 22 is configured to rotate with respect to the plate 21 so as to make the connection of the connector 11 to the counter-connector 12a-c stable.

The rotation of the seating 22 can be effected by means of a corresponding drive lever 25, which can be driven manually by an operator so that the pins 23 are positioned on the bottom of the corresponding slits 24.

The connection head 17 able to engage in said seating 22 thus represents, as we said, one of the various removable connection means of the connector 11 to the counter-connector 12a-c.

On the machine side, the counter-connector 12a-c has counter-connection elements 26 to allow the passage of a determinate treatment fluid according to the treatment step of the medical instrument 16.

This passage can be univocal, or also provide a return.

Each of the counter-connection elements 26 is able to engage fluidically, hence to be removably connected to a corresponding connection element 18 of the connector 11.

As we said, the medical instrument 16 can be an endoscope provided with a flexible tube 27 that connects a control and service unit 28 and an operating unit 29.

Inside the flexible tube 27, all the channels or connection elements between the two units 28 and 29 are collected. The channels can be used, for example, for functions of conduction and/or aspiration of sterile or contaminated substances, to accommodate connections for optical or surgical devices or other.

Each of the connection elements or channels of the medical instrument 16 is connected to the tubular elements 20 of the connector 11 by means of suitable removable connection means. This step is shown schematically in fig. 2a.

The connector 11 with the medical instrument 16 connected can be put in a suitable container, for example a rack, where the medical instrument 16 is housed in a predefined position at least with its connector. Advantageously, according to some possible embodiments, from now on and until the end of the treatment cycle, only the rack will be moved, transported and handled, while the medical instrument 16 is no longer handled, remaining connected and protected by the connector 11.

According to other variants, the medical instrument 16, being always connected to the connector 11 in the passage between one reconditioning step and the other, can also be handled by an operator in terms of movement and/or transport between one machine and the other and/or in terms of positioning or housing in a desired reconditioning machine. However, thanks to the present invention, the operator will no longer be required to connect and disconnect the medical instrument 16 from the connector 11, since these two elements will always remain connected during the reconditioning cycle and in the passage from one intermediate step to the other, thus isolating the medical instrument 16, and advantageously the apertures, or terminals thereof, from the risk of contamination. In practice therefore, the connector 11, being always connected to the medical instrument 16 from the beginning to the end of the conditioning cycle, during the intermediate reconditioning steps and in the passage between them, protects the medical instrument 16 from contamination, and in particular protects the apertures or terminals thereof, since the latter are always connected to the tubular elements 20 of the connector 11 and hence isolated from the outside.

At the end of this step of univocal connection of the tubular elements 20 to the connection elements, channels or terminals of the medical instrument 16, for example using the rack or being positioned manually, the connector 11, which is connected on the instrument side, is then associated, on the machine side, with the counter-connector 12a of the machine 13, so that the medical instrument 16 can be subjected, for example, to a first pre-washing step, in which the tubular elements 20 are also advantageously prewashed inside.

The connection between the connector 11 already connected on the instrument side and the counter-connector 12a on the machine side is carried out by inserting the connection head 17 inside the seating 22 of the counter-connector 12a in a sealed manner, so that the connection elements 18 are connected to the corresponding counter-connection elements 26.

At the end of the prewash step, where the device or machine concerned is only dedicated to the prewash, the connector 11, always held connected with the medical instrument 16, is rapidly and effectively removed from the counter-connector 12a of the prewash machine 13.

At this point, the rack containing the connector 11 connected to the medical instrument 16 that has been subjected to the prewash step, or directly the medical instrument 16 connected to the connector 11 if the rack is not provided, is inserted into a washing and disinfection machine 14, advantageously without needing to re-connect the various channels of the medical instrument 16 one by one to the machine 14, since, thanks to the fact that the connector 11 is already connected with its tubular elements 20 to the apertures or terminals of the medical instrument 16, it is sufficient to connect the machine side connector 11 with a single standardized operation. In fact, the machine-side connection operation is carried out simply and quickly by inserting the connector 11 into the counter-connector 12b of the machine 14, in a similar way to that described for the machine 13, therefore by means of a sealed insertion of the connection head 17 in the seating 22 of the counter-connector 12b, so that the connection elements 18 are again connected to the corresponding counter-connection elements 26. As we said, in some possible variants, the medical instrument 16 is in no way handled, but left in the rack, while in other variants it can be moved by an operator directly without using a rack, in both cases with the provision that the same medical instrument 16 will still be always connected to the connector 11, which therefore protects it and isolates it from the risk of contamination.

At the end of this washing and disinfection step inside the machine 14, in which advantageously the tubular elements 20 and the apertures or terminals of the medical instrument 16 are then also washed and disinfected inside, by means of suitable fluids and/or treatment systems, the connector 11 is disengaged from the counter-connector 12b and hence another treatment step of the medical instrument 16 can be performed in another machine 15 as described below in detail, that is to say, a drying and storage step, the latter generally carried out in a machine 15 which is a drying and storage apparatus, also called drying and storage cabinet, where the medical instrument 16 can be dried and stored under sterile conditions until required for a new use.

The drying and storage step is carried out by moving the rack, containing inside it the connector 11 with the medical instrument 16 connected, into the machine 15, where the rack is deposited, or by directly transporting the medical instrument 16, without using the rack, and housing it inside the machine 15, for example, deposited horizontally, hanging vertically, in an elongated/extended configuration, or wound or rolled or otherwise, as needed. The connector 11, always connected on the instrument side, is connected on the machine side to the counter-connector 12c of the drying and storage machine 15, by means of a new sealed insertion of the connection head 17 into the seating 22 of the counter-connector 12c of the machine 15, so that the connection elements 18 are again connected to the corresponding counter-connection elements 26.

At the end of this step carried out in the machine 15, in which the tubular elements 20 and the apertures or terminals of the medical instrument 16 are advantageously also dried inside, the connector 11 and the medical instrument 16 can be stored in a connected condition in the machine 15 itself, until required for a new use, or can be disconnected and separated in the event of a new use, as shown schematically in fig. 2c, that is to say, the tubular elements 20 of the connector 11 can be univocally separated from the channels or connections of the medical instrument 16 on which a complete reconditioning cycle has been carried out, to be used in a new use.

In particular, since the machine 15 can typically be used for both drying and storing/preserving reconditioned medical instruments 16, until required for a new use, the disconnection can be performed at the time when a medical instrument 16 is removed from the machine 15 for a new use, thus guaranteeing sterility until before said new use.

The medical instrument 16 has therefore been subjected to a complete reconditioning cycle, advantageously by a single connection operation, at the beginning of the reconditioning cycle, and disconnection, at the end of the reconditioning cycle when the instrument 16 has to be used again, of the corresponding channels or internal connections with the tubular elements 20 of the connector 11.

The connection of the tubular elements 20 of the connector 11 is therefore only made prior to the entrance into the first treatment machine 13, whereas the disconnection is made only at the end of the reconditioning cycle, as required, when it is necessary to remove the medical instrument 16 from the machine 15 for a new use of the medical instrument 16 itself, dried and stored/preserved in sterile conditions, contained in a rack in a container, in a drawer or case, or free, but still inside the machine 15.

The univocal operations to connect and disconnect the medical instrument 16 to the connector 11 naturally reduce the risk of contamination of the medical instrument 16 and also reduce working times, making the treatment cycle faster and more efficient.

The medical instrument 16 is therefore handled only at the beginning of the treatment cycle to perform a univocal operation to connect the various channels with the connector and at the end of the cycle to disconnect.

According to possible embodiments of the method described here, in the intermediate steps of the cycle, for the movements from one machine 13, 14 and 15 to another, only the rack or other housing container of the medical instrument 16 and the connector 11, in some variants, are handled, but the medical instrument 16 itself is not handled, at least in terms of connection or disconnection with respect to the connector 11. Alternatively, in other variants of the method, the medical instrument 16 can also be handled directly, in order to transport it or position it in relation to the various machines 13, 14 and 15, but not in terms of connection and disconnection with respect to the connector 11, which is always connected during the reconditioning cycle and in the passage from one step to another, thus isolating as much as possible the medical instrument 16 and the apertures, or terminals, thereof, from the risk of contamination. It is also possible that, for some movements or positioning in relation to one or more of the machines 13, 14 and 15, a suitable rack, drawer, case, or similar container is used, and for other movements the medical instrument 16, always connected to the connector 11, is handled directly to be moved or positioned in relation to one or more of the machines 13, 14 and 15.

The connection shown between the connection head 17 and the seating 22 of any of the counter-connectors 12a, 12b or 12c is extremely precise, therefore the possibility of coupling errors of the connector to the counter-connector is drastically reduced.

The connection of the connector 11 to any one of the counter/connectors 12a, 12b or 12c is also very fast and allows a univocal identification that simplifies the monitoring of the reconditioning cycle.

The operations performed on the connector 11 during the reconditioning cycle or the complete treatment of the medical instrument 16 are also advantageously facilitated by the ergonomic shape of the connector 11 itself.

It is clear that modifications and/or additions of parts may be made to the method and system for reconditioning a medical instrument, in particular an endoscope, as described heretofore, without departing from the field and scope of the present invention.

It is also clear that, although the present invention has been described with reference to some specific examples, a person of skill in the art shall certainly be able to achieve many other equivalent forms of method and system for performing a reconditioning cycle on a medical instrument, in particular an endoscope, having the characteristics as set forth in the claims and hence all coming within the field of protection defined thereby.

In the following claims, the sole purpose of the references in brackets is to facilitate reading: they must not be considered as restrictive factors with regard to the field of protection claimed in the specific claims.

## Claims

1. A system of reconditioning a medical instrument (16) comprising a device (10) and distinct dedicated reconditioning machines (13, 14, 15) to recondition said medical instrument (16), said device (10) comprising a single connector (11) configured to be removably connected to the medical instrument (16) and to counter-connectors (12a, 12b, 12c) of said distinct dedicated reconditioning machines (13, 14, 15), said connector (11) and said counter-connector (12a, 12b, 12c) being mating,
wherein said connector (11) comprises a connection head (17) provided with a series of connection elements (18) and on one side a plurality of tubular elements (20),
wherein a specific tubular treatment element (20) is connected to each of the connection elements (18), inside a casing (19) of the connector (11), in which tubular treatment element (20) fluid for washing or disinfection can flow,
wherein each of the tubular elements (20) is flexible and is connectable by means of suitable connection means to apertures or inlets of the medical instrument (16), so as to reach the channels and the internal zones,
wherein the counter-connector (12a, 12b, 12c) is installed on each of the machines (13, 14, 15) and comprises a support plate (21) which is connectable to one of the walls of the machine (13, 14, 15) and a seating (22) and
wherein the connection head (17) of the connector (11) is inserted into the seating (22) of the counter-connector (12a, 12b, 12c), so as to obtain a stable and sealed connection.

2. System as in claim 1, **characterized in that** said connector (11) comprises on one side said plurality of tubular elements (20), able to be univocally connected to said medical instrument (16) from the beginning to the end of the reconditioning cycle, and on the other side comprises said connection elements (18) able to be connected to corresponding counter-connection elements (26) provided in each machine (13, 14, 15) of the reconditioning cycle.

3. System as in claim 1 or 2, **characterized in that** an intermediate connector element is provided between said connector (11) and said counter-connector (12a, 12b, 12c).

4. Method to recondition a medical instrument (16) by means of a system as in any claim herein before, the method comprising:
- a first step of univocally connecting said medical instrument (16) to a connector (11);
- intermediate steps of reconditioning said medical instrument (16) in which said connector (11), with said medical instrument (16) connected, is associated with a corresponding reconditioning counter-connector (12a, 12b, 12c); said intermediate reconditioning steps of said medical instrument (16) are carried out in succession inside respective and distinct dedicated reconditioning machines (13, 14, 15), each provided with its own and respective counter-connector (12a, 12b, 12c), said method providing to keep said connector (11) always connected to said medical instrument (16) during the course of said intermediate reconditioning steps and also during the passage from one intermediate step to another, and, on each occasion, in relation to the intermediate reconditioning steps that follow each other, to connect/disconnect said single specific connector (11) to/from a respective counter-connector (12a, 12b, 12c) of the specific reconditioning machine (13, 14, 15) in which a respective specific intermediate reconditioning step is carried out, wherein said connector (11) comprises a connection head (17) provided with a series of connection elements (18) and on one side a plurality of tubular elements (20), wherein a specific tubular treatment element (20) is connected to each of the connection elements (18), inside a casing (19) of the connector (11), in which tubular treatment element (20) fluid for washing or desinfection can flow, wherein each of the tubular elements (20) is flexible and is connectable by means of suitable connection means to apertures or inlets of the medical instrument (16), so as to reach the channels and the internal zones, wherein the counter-connector (12a, 12b, 12c) is installed on each of the machines (13, 14, 15) and comprises a support plate (21) which is connectable to one of the walls of the machine (13, 14, 15) and a seating and wherein the connection head (17) of the connector (11) is inserted into the seating (22) of the counter-connector (12a, 12b, 12c), so as to obtain a stable and sealed connection;
- a final step of univocally disconnecting said connector (11) from said medical instrument (16) once the reconditioning cycle has been completed, before said medical instrument (16) is again used.

5. Method as in claim 4, **characterized in that** during said intermediate reconditioning steps, said connector (11) with said medical instrument (16) connected is housed and positioned in a defined manner in a suitable movement container, such as a rack.

6. Method as in claim 4 or 5, **characterized in that** said intermediate reconditioning steps of said medical instrument (16) comprise at least a prewashing step, at least a washing and disinfecting step and at least a drying and storage step.

## Patentansprüche

1. System zur Wiederinstandsetzung eines medizinischen Instrumentes (16) umfassend eine Vorrichtung (10) und verschiedene zugeordnete Wiederinstandsetzungsmaschinen (13, 14, 15) zur Wiederinstandsetzung des genannten medizinischen Instrumentes (16), wobei die genannte Vorrichtung (10) einen einzelnen Verbinder (11) umfasst, der dafür ausgelegt ist, mit dem medizinischen Instrument (16) und mit Gegen-Verbindern (12a, 12b, 12c) der genannten verschiedenen zugeordneten Wiederinstandsetzungsmaschinen (13, 14, 15) abnehmbar verbunden zu werden, wobei der genannte Verbinder (11) und der genannte Gegen-Verbinder (12a, 12b, 12c) zusammenpassen,
worin der genannte Verbinder (11) einen Verbindungskopf (17), der mit einer Reihe von Verbindungselementen (18) versehen ist, und an einer Seite eine Vielzahl von rohrförmigen Elementen (20) umfasst,
worin ein spezielles rohrförmiges Aufbereitungselement (20) mit jedem der Verbindungselemente (18), innerhalb eines Gehäuses (19) des Verbinders (11), verbunden ist, in welches rohrförmige Aufbereitungselement (20) eine Flüssigkeit zum Waschen oder zur Desinfektion fließen kann,
worin jedes der rohrförmigen Elemente (20) flexibel ist und mittels geeigneter Verbindungsmittel mit Öffnungen oder Einlässen des medizinischen Instrumentes (16) verbindbar ist, so dass die Kanäle und die inneren Bereiche erreicht werden,
worin der Gegen-Verbinder (12a, 12b, 12c) an jeder der Maschinen (13, 14, 15) installiert ist und eine Stützplatte (21), die mit einer der Wände der Maschine (13, 14, 15) verbindbar ist, und einen Sitz (22) umfasst, und
worin der Verbindungskopf (17) des Verbinders (11) in den Sitz (22) des Gegen-Verbinders (12a, 12b, 12c) eingefügt ist, um eine stabile und abgedichtete Verbindung zu erhalten.

2. System nach Anspruch 1, **dadurch gekennzeichnet, dass** der genannte Verbinder (11) an einer Seite die genannte Vielzahl von rohrförmigen Elementen (20), die mit dem genannten medizinischen Instrument (16) vom Anfang bis zum Ende des Wiederinstandsetzungszyklus eindeutig verbunden werden können, und an der anderen Seite die genannten Verbindungselemente (18) umfasst, die mit entsprechenden Gegen-Verbindungselementen (26) verbunden werden können, die in jeder Maschine (13, 14, 15) des Wiederinstandsetzungszyklus vorgesehen sind.

3. System nach Anspruch 1 oder 2, **dadurch gekennzeichnet, dass** ein Zwischenverbinderelement zwischen dem genannten Verbinder (11) und dem genannten Gegen-Verbinder (12a, 12b, 12c) vorgesehen ist.

4. Verfahren zur Wiederinstandsetzung eines medizinischen Instrumentes (16) mittels eines Systems nach einem der vorhergehenden Ansprüche, wobei das Verfahren umfasst:
- einen ersten Schritt des eindeutigen Verbindens des genannten medizinischen Instrumentes (16) mit einem Verbinder (11);
- Zwischenschritte des Wiederinstandsetzens des genannten medizinischen Instrumentes (16), in denen der genannte Verbinder (11), mit dem verbundenen genannten medizinischen Instrument (16), mit einem entsprechenden Wiederinstandsetzungs-Gegen-Verbinder (12a, 12b, 12c) verbunden wird; die genannten Zwischenwiederinstandsetzungsschritte des genannten medizinischen Instrumentes (16) in Reihenfolge innerhalb von jeweiligen und verschiedenen zugeordneten Wiederinstandsetzungsmaschinen (13, 14, 15) durchgeführt werden, die je mit ihrem eigenen und jeweiligen Gegen-Verbinder (12a, 12b, 12c) versehen sind, wobei das genannte Verfahren vorsieht, den genannten Verbinder (11) während des Ablaufs der genannten Zwischenwiederinstandsetzungsschritte und auch während des Übergangs von einem Zwischenschritt zum anderen immer mit dem genannten medizinischen Instrument (16) verbunden zu halten und, jedesmal, in Bezug auf die Zwischenwiederinstandsetzungsschritte, die aufeinander folgen, den genannten einzelnen speziellen Verbinder (11) mit/von einem jeweiligen Gegen-Verbinder (12a, 12b, 12c) der speziellen Wiederinstandsetzungsmaschine (13, 14, 15) zu verbinden/zu trennen, in welcher ein jeweiliger spezieller Zwischenwiederinstandsetzungsschritt durchgeführt wird, worin der genannte Verbinder (11) einen Verbindungskopf (17), der mit einer Reihe von Verbindungselementen (18) versehen ist, und an einer Seite eine Vielzahl von rohrförmigen Elementen (20) umfasst, worin ein spezielles rohrförmiges Aufbereitungselement (20) mit jedem der Verbindungselemente (18), innerhalb eines Gehäuses (19) des Verbinders (11), verbunden ist, in welches rohrförmige Aufbereitungselement (20) eine Flüssigkeit zum Waschen oder zur Desinfektion fließen kann, worin jedes der rohrförmigen Elemente (20) flexibel ist und mittels geeigneter Verbindungsmittel mit Öffnungen oder Einlässen des medizinischen Instrumentes (16) verbindbar ist, so dass die Kanäle und die inneren Bereiche erreicht werden, worin der Gegen-Verbinder (12a, 12b, 12c) an jeder der Maschinen (13, 14, 15) installiert ist und eine Stützplatte (21), die mit einer der Wände der Maschine (13, 14, 15) verbindbar ist, und einen Sitz umfasst, und worin der Verbindungskopf (17) des Verbinders (11) in den Sitz (22) des Gegen-Verbinders (12a, 12b, 12c) eingefügt ist, um eine stabile und abgedichtete Verbindung zu erhalten;
- einen Endschritt des eindeutigen Trennens des genannten Verbinders (11) von dem genannten medizinischen Instrument (16), nachdem der Wiederinstandsetzungszyklus abgeschlossen wurde, bevor das genannte medizinische Instrument (16) wieder benutzt wird.

5. Verfahren nach Anspruch 4, **dadurch gekennzeichnet, dass** während der genannten Zwischenwiederinstandsetzungsschritte der genannte Verbinder (11) mit dem verbundenen genannten medizinischen Instrument (16) in definierter Weise in einem geeigneten Bewegungsbehälter, wie einem Gestell, beherbergt und positioniert ist.

6. Verfahren nach Anspruch 4 oder 5, **dadurch gekennzeichnet, dass** die genannten Zwischenwiederinstandsetzungsschritte des genannten medizinischen Instrumentes (16) zumindest einen Vorwaschschritt, zumindest einen Wasch- und Desinfektionsschritt und zumindest einen Trocken- und Lagerungsschritt umfassen.

## Revendications

1. Système de reconditionnement d'un instrument médical (16) comprenant un dispositif (10) et des machines de reconditionnement dédiées distinctes (13, 14, 15) pour reconditionner ledit instrument médical (16), ledit dispositif (10) comprenant un seul connecteur (11) configuré pour être connecté de manière amovible à l'instrument médical (16) et à des contre-connecteurs (12a, 12b, 12c) desdites machines de reconditionnement dédiées distinctes (13, 14, 15), ledit connecteur (11) et ledit contre-connecteur (12a, 12b, 12c) étant appariés,
dans lequel ledit connecteur (11) comprend une tête de connexion (17) pourvue d'une série d'éléments de connexion (18) et d'un côté une pluralité d'éléments tubulaires (20),
dans lequel un élément de traitement tubulaire spécifique (20) est connecté à chacun des éléments de connexion (18), à l'intérieur d'un boîtier (19) du connecteur (11), dans lequel l'élément de traitement tubulaire (20) un fluide pour de lavage ou de désinfection peut circuler,
dans lequel chacun des éléments tubulaires (20) est flexible et peut être raccordé, par des moyens de connexion appropriés, à des ouvertures ou des entrées de l'instrument médical (16), afin d'atteindre les canaux et les zones internes,
dans lequel le contre-connecteur (12a, 12b, 12c) est installé sur chacune des machines (13, 14, 15) et comprend une plaque de support (21) qui peut être reliée à l'une des parois de la machine (13, 14, 15) et un siège (22), et
dans lequel la tête de connexion (17) du connecteur (11) est insérée dans le siège (22) du contre-connecteur (12a, 12b, 12c), afin d'obtenir une connexion stable et étanche.

2. Système selon la revendication 1, **caractérisé en ce que** ledit connecteur (11) comprend d'un côté ladite pluralité d'éléments tubulaires (20), aptes à être connectés de manière univoque audit instrument médical (16) du début à la fin du cycle de reconditionnement, et comprend de l'autre côté lesdits éléments de connexion (18) pouvant être connectés à des éléments de contre-connexion correspondants (26) prévus dans chaque machine (13, 14, 15) du cycle de reconditionnement.

3. Système selon la revendication 1 ou 2, **caractérisé en ce qu'**un élément de connexion intermédiaire est prévu entre ledit connecteur (11) et ledit contre-connecteur (12a, 12b, 12c).

4. Procédé de reconditionnement d'un instrument médical (16) au moyen d'un système selon l'une quelconque des revendications précédentes, ce procédé comprenant :
• une première étape de connexion univoque dudit instrument médical (16) à un connecteur(11) ;
• des étapes intermédiaires de reconditionnement dudit instrument médical (16) dans lesquelles ledit connecteur (11), avec ledit instrument médical (16) connecté, est associé à un contre-connecteur de reconditionnement correspondant (12a, 12b, 12c) ; lesdites étapes intermédiaires de reconditionnement dudit instrument médical (16) sont effectuées successivement à l'intérieur de machines de reconditionnement dédiées, respectives distinctes (13, 14, 15), chacune pourvue de son propre contre-connecteur respectif (12a, 12b, 12c), ledit procédé prévoyant de maintenir ledit connecteur (11) toujours connecté audit instrument médical (16) au cours desdites étapes intermédiaires de reconditionnement ainsi que lors du passage d'une étape intermédiaire à une autre, et, à chaque fois, en fonction des étapes intermédiaires de reconditionnement qui se succèdent, de connecter/déconnecter ledit connecteur spécifique unique (11) à/de un contre-connecteur respectif (12a, 12b, 12c) de la machine de reconditionnement spécifique (13, 14, 15) dans laquelle une étape de reconditionnement intermédiaire spécifique respective est effectuée, dans lequel ledit connecteur (11) comprend une tête de connexion (17) munie d'une série d'éléments de connexion (18) et, d'un côté, d'une pluralité d'éléments tubulaires (20), où un élément de traitement tubulaire spécifique (20) est connecté à chacun des éléments de connexion (18), à l'intérieur d'un boîtier (19) du connecteur (11), élément de traitement tubulaire spécifique (20) dans lequel un fluide de lavage ou de désinfection peut s'écouler, dans lequel chacun des éléments tubulaires (20) est flexible et peut être connecté par des moyens de connexion appropriés à des ouvertures ou des entrées de l'instrument médical (16), de manière à atteindre les canaux et les zones internes, dans lequel le contre-connecteur (12a, 12b, 12c) est installé sur chacune des machines (13, 14, 15) et comprend une plaque de support (21) qui peut être raccordée à l'une des parois de la machine (13, 14 15) et un siège et où la tête de connexion (17) du connecteur (11) est insérée dans le siège (22) du contre-connecteur (12a, 12b, 12c), de manière à obtenir une connexion stable et étanche ;
• une étape finale de déconnexion univoque dudit connecteur (11) dudit instrument médical (16) une fois le cycle de reconditionnement terminé, avant que ledit instrument médical (16) ne soit à nouveau utilisé.

5. Procédé selon la revendication 4, **caractérisé en ce que** pendant les étapes intermédiaires de reconditionnement, ledit connecteur (11) avec ledit instrument médical (16) connecté est logé et positionné de manière définie dans un conteneur de déplacement approprié, tel qu'un rack.

6. Procédé selon la revendication 4 ou 5, **caractérisé en ce que** lesdites étapes intermédiaires de reconditionnement dudit instrument médical (16) comprennent au moins une étape de prélavage, au moins une étape de lavage et de désinfection et au moins une étape de séchage et de stockage.
